# EUROPÄISCHE PATENTSCHRIFT

(11) **EP 3 275 430 B1**
(45) Veröffentlichungstag und Bekanntmachung des Hinweises auf die Patenterteilung: **21.06.2023**
(21) Anmeldenummer: 17165443.7
(22) Anmeldetag: 07.04.2017
(51) Int. Cl.: A61K 47/24, A61K 31/12, A61K 33/04

(54) **CURCUMINLÖSUNG ZUR INTRAVENÖSEN INFUSION**
CURCUMIN SOLUTION FOR INTRAVENOUS INFUSION
SOLUTION DE CURCUMINE POUR INFUSION INTRAVEINEUSE

(43) Veröffentlichungstag der Anmeldung: 31.01.2018
(73) Patentinhaber: Fernando, Rohan Charles, 69120 Heidelberg (DE)
(72) Erfinder: Fernando, Rohan Charles, 69120 Heidelberg (DE)
(74) Vertreter: Zellentin & Partner mbB Patentanwälte

(56) Entgegenhaltungen:
- WO-A1-2011/101859
- WO-A1-2014/063844
- K. SHAHANI ET AL: "Injectable Sustained Release Microparticles of Curcumin: A New Concept for Cancer Chemoprevention", CANCER RESEARCH, Bd. 70, Nr. 11, 11. Mai 2010 (2010-05-11), Seiten 4443-4452, XP055404553, US ISSN: 0008-5472, DOI: 10.1158/0008-5472.CAN-09-4362
- WU XUEMEI ET AL: "Self-microemulsifying drug delivery system improves curcumin dissolution and bioavailability", DRUG DEVELOPMENT AND INDUSTRIAL PHARMACY ENG, INFORMA HEALTHCARE, US, Bd. 37, Nr. 1, 1. Januar 2011 (2011-01-01) , Seiten 15-23, XP009173241, ISSN: 1520-5762, DOI: 10.3109/03639045.2010.489560

## Beschreibung

Die vorliegende Erfindung betrifft eine Lösung zur intravenösen Infusion enthaltend Curcumin sowie ein Konzentrat zur Herstellung der Lösung.

Curcumin ist ein Inhaltsstoff des unter der Bezeichnung Curry bekannten Gewürzes, welches in der indischen Küche viel verwendet wird. Seit längerem zeigen Untersuchungen, dass Curcumin bei einer ganzen Reihe Erkrankungen positive Wirkungen hat, siehe z.B. S.C. Gupty et al., "Therapeutic roles of curcumin: lessons learned from clinical trials", AAPS Journal Vol. 15, No. 1, pp. 195-218, January 2013, und P. Anand et al., "Curcumin and cancer: an "old-age" disease with an "age-old" solution", Canc. Lett. Vol 267 (2008), pp. 133-164. Überwiegend wird über eine orale Verabreichung berichtet, in einigen Fällen auch über eine topische oder intravenöse Dosierung.

Wegen der geringen Bioverfügbarkeit von oral dosiertem Curcumin wurden auch schon Lösungen für die intravenöse Dosierung vorgeschlagen. So beschreibt US 2015/0342904 A1 eine Formulierung, bei der Curcumin in Alkohol gelöst und dann mit einer Säure und einem Lösungsvermittler versetzt wird. Die lagerstabile Formulierung kann mit Wasser zu einer Infusionslösung gemischt werden. Gemäß einem anderen Vorschlag in WO 2015/077640 A1 wird Curcumin in Liposomen auf Basis von Lipiden oder Phospholipiden eingekapselt, die dann in eine wässrige Infusionslösung eingebracht werden können. Die Artikel K. Shahani et al., "Injectable sustained release microparticles of curcumin: a new concept for cancer chemoprevention", Cancer Research Bd. 70, Nr. 11, 2010, S. 4443-4452 und X. Wu et al., "Self-microemulsifying drug delivery system improves curcumin dissolution and bioavailability", Drug Developm. Industr. Applica. Bd. 37, Nr. 1, 2001, S. 15-23, sowie WO 2011/101859 A1 beschreiben zu Liposomen analoge Ansätze. K. Shahani et al. schlagen Mikropartikel aus Poly(D,L-lactid-co-glycolid) vor, X. Wu et al. beschreiben eine selbst emulgierende Darreichungsform bestehend aus Wirkstoff, Öl, Tensid und Cotensid, die vorsichtig gemischt werden um Mikroemulsionströpfchen zu bilden, und WO 2011/101859 A1 betrifft Nanopartikel aus Glycerolmonooleat, Polyvinylalkohol und Pluronic F-127. Beide Ansätze, Mischung mit Alkohol/Lösungsvermittler und Liposomen, sind nicht ideal. Die Herstellung von Liposomen ist technologisch aufwendig und für eine pharmakologische Wirkung muss das Curcumin im Körper erst wieder aus den Liposomen freigesetzt werden. Alkohol ist bei einer intravenösen Infusion schlecht verträglich. In diesem Kontext werden in gängiger Praxis bei Alkohol-haltigen Curcumin Formulierungen Infusionsvorläufe mit beispielsweise Antiemetika, Kortison, Antiallergika und Antazida, um die Infusionsverträglichkeit zu erhöhen, verabreicht. Die Infusion muss trotzdem sehr langsam erfolgen und darüber hinaus ergibt sich ein nicht zu vernachlässigender Blutalkoholspiegel.

Es besteht daher weiter die Aufgabe, eine Möglichkeit der Applikation von Curcumin zu finden, bei welcher sowohl eine gute Bioverfügbarkeit als auch eine gute Verträglichkeit gewährleistet sind.

Überraschend wurde nun gefunden, dass sich eine zur i.v. Infusion geeignete wässrige Lösung von Curcumin erhalten lässt, wenn man Curcumin mit Dimethylsulfoxid und einem Lösungsvermittler vermischt und das Gemisch unter Zusatz von einem Puffer und Natriumselenit in einem wässrigen Infusionsmedium wie beispielsweise steriles Wasser (*Aqua ad injectabilia*) isotonische Kochsalzlösung und 5 %-ige Glukoselösung einbringt.

Die obige Aufgabe wird daher durch eine wässrige Infusionslösung gelöst, die Curcumin und/oder ein oder mehrere Curcuminderivat(e), nämlich Demethoxycurcumin und/oder Bisdemethoxycurcumin, Dimethylsulfoxid, mindestens einen Lösungsvermittler, einen Puffer und Natriumselenit in einem wässrigen Infusionsmedium enthält. Die Aufgabe wird außerdem durch ein Konzentrat gemäß Anspruch 13 zur Herstellung einer Infusionslösung gelöst, welches Curcumin und/oder oder mehrere Curcuminderivat(e), Dimethylsulfoxid, mindestens einen Lösungsvermittler und Natriumselenit enthält.

Durch Verdünnen des Konzentrats mit einem wässrigen Infusionsmedium unter Zusatz eines Puffers erhält man die erfindungsgemäße Infusionslösung. Diese enthält sofort verfügbares Curcumin bzw. Curcuminderivat und ist frei von Alkohol mit seinen Problemen. Die Infusionsgeschwindigkeit kann daher im Vergleich zu Alkohol enthaltenden Lösungen viel höher sein, weil keine Reizungen der Gefäße durch Alkohol zu befürchten sind. Curcumin bzw. sein Derivat gelangt unmittelbar in den Blutkreislauf, ohne Verzögerung bis zur Freisetzung aus Liposomen.

Curcumin und Curcuminderivate, welche für therapeutische Zwecke inklusive zur Infusion geeignet sind, sind bekannt, siehe z.B. die o.g. Patentanmeldungen und auch die darin zitierte Literatur. Bevorzugt wird Curcumin extrahiert aus biologisch (d.h. ohne chemische Dünger, Pestizide, Herbizide und Insektizide) angebautem Kurkuma (*Curcuma longa*) verwendet. Aber auch natürliche und synthetische Curcuminderivate wie sie z.B. im o.g. Stand der Technik beschrieben wurden sind brauchbar. Im folgenden werden Curcumin und Curcuminderivate auch zusammenfassend als Curcumin(derivat) bezeichnet, wobei damit sowohl Curcumin als auch ein oder mehrere Curcuminderivate als auch ein Gemisch von Curcumin mit einem oder mehreren Curcuminderivat(en) gemeint ist.

Dimethylsulfoxid, kurz DMSO, ist als Inhaltsstoff von topischen Arzneimitteln bekannt. Es ist in geringen Dosierungen gut verträglich. Gemäß G. Da Violante et al., "Evaluation of the cytotoxicity effect of dimethyl sulfoxide (DMSO) on Caco2/TC7 colon tumor cell cultures", Biol Pharm Bull. Vol. 25(12), pp.1600-1603, Dec. 2002 vertrugen Zellen problemlos bis zu 10 %-ige DMSO Lösungen. Solche Konzentrationen werden bei der Infusion der erfindungsgemäßen Infusionslösungen bei weitem nicht erreicht.

Als Lösungsvermittler werden bevorzugt solubilisierende Polymere ausgewählt, wie Polyvinylpyrrolidon, Addukte von Ethylenoxid oder Propylenoxid an physiologisch verträgliche Öle, Polyethylenglykole und Polysorbate. Besonders bevorzugt sind Polyethylenglykole und Polysorbate sowie Gemische davon. Geeignete Lösungsvermittler sind kommerziell erhältlich, beispielsweise unter der Bezeichnung Kolliphor von BASF SE, DE. Mit der Bezeichnung Lösungsvermittler sind im Rahmen der vorliegenden Anmeldung sowohl Einzelsubstanzen als auch Gemische von zwei oder mehr Lösungsvermittlern gemeint, soweit nichts anderes angegeben ist.

Das wässrige Infusionsmedium, im folgenden auch Infusionsmedium, ist vorzugsweise steriles Wasser (Aqua ad injectabilia), isotonische Kochsalzlösung oder Glukoselösung. Die Glukoselösung wird insbesondere als 5 %-ige Lösung eingesetzt. Andere physiologisch verträgliche Flüssigkeiten und Lösungen sind ebenfalls mögliche Infusionsmedien, sofern sie mit DMSO mischbar sind und keine Veränderung des Curcumin(derivat)s verursachen. Auch Gemische verschiedener Infusionsmedien sind geeignet.

Ein wichtiger Bestandteil der erfindungsgemäßen Infusionslösung ist Natriumselenit, Na₂SeO₃. Natriumselenit allein oder im Gemisch mit ein oder mehreren weiteren Antioxidantien ist ein potenter antioxidativer und antientzündlicher Inhaltsstoff. In Kombination mit Curcumin, das ebenfalls ausgesprochen antioxidative und antientzündliche Eigenschaften aufweist, ist eine synergistische Wirkung zu erkennen. Im Kontext des oxidativen Stresses (Überangebot an freien Radikalen) und entzündlichen Prozessen spielt die Aktivierung des sog. Transkriptionsfaktors, NF-kappaB eine Schlüsselrolle. Curcumin und Selen (Natriumselenit) sind selektive Hemmstoffe der Aktivierung jenes Transkriptionsfaktors. Bei Zugabe von Natriumselenit geschieht die vermittelte Unterdrückung bereits unter physiologischen Konzentrationen. F. Maehira et al., Clin Chim Acta, 2003 Aug; 334 (1-2): 163-171, "Selenium regulates transcription factor NF-kappaB activation during the acute phase reaction".

Ein weiterer Vorteil des Natriumselenits für die erfindungsgemäße Formulierung ist die Erhöhung der Stabilität des Curcumin(derivat)s in wässriger Lösung. Prinzipiell können zusätzlich weitere Antioxidantien, wie beispielsweise Ascorbinsäure, N-Acetylcystein und Glutathion eingesetzt werden.

Besonders bevorzugt wird Na₂SeO₃ bereits dem dann erfindungsgemäßen Konzentrat zugefügt. Es ist aber auch möglich, ein Konzentrat ohne Natriumselenit herzustellen und Natriumselenit erst der Infusionslösung zuzufügen.

Das erfindungsgemäße Konzentrat enthält zumindest Curcumin(derivat), Lösungsvermittler, Natriumselenit und DMSO. Zu seiner Herstellung wird zweckmäßig zunächst Curcumin(derivat), üblicherweise bei Raumtemperatur, in sterilem DMSO gelöst. Im weiteren Schritt wird der Lösungsvermittler dem gelösten Curcumin(derivat) unter Rühren zugegeben. Sofern der Lösungsvermittler im festen Aggregatzustand vorliegt, wird er zuvor durch Erwärmen, z.B. auf etwa 50°C bis 60°C, verflüssigt. Vorzugsweise wird in Infusionsmedium gelöstes Natriumselenit, hinzugefügt. Es wird, ggfs. nach dem raschen Abkühlen auf Raumtemperatur, ein nahezu wasserfreies, lagerstabiles pastöses Konzentrat erhalten.

Brauchbare Mengen Lösungsvermittler liegen im Bereich von 15 g bis 25 g pro 1 g Curcumin(derivat), vorzugsweise 20 g bis 30 g. Brauchbare Mengen DMSO liegen im Bereich von 2 g bis 6 g pro 1 g Curcumin(derivat), vorzugsweise 2 g bis 4 g. Sofern Natriumselenit allein oder im Gemisch mit weiteren Antioxidantien dem Konzentrat zugemischt wird, werden Mengen von 0,1 bis 0,6 mg pro 1 g Curcumin(derivat), vorzugsweise von 0,1 bis 0,25 mg eingesetzt.

Ein großer Vorteil des erfindungsgemäßen Konzentrats ist seine Lagerstabilität. Die Lagerstabilität kann erfindungsgemäß 24 Monate und mehr betragen.

Ein weiterer Vorteil des erfindungsgemäßen Konzentrats ist die leichtere Handhabung beim Abwiegen des stabilisierten Konzentrats.

Außerdem und vor allem ergibt sich eine erleichterte Herstellung der parenteral zu verabreichenden Infusionslösung. Als Infusionsmedium können z.B. steriles Wasser, isotonische Kochsalzlösung oder andere für Infusionszwecke geeignete Lösungen verwendet werden. Bei der erfindungsgemäßen Infusionslösung liegt das aus dem Konzentrat gelöste Curcumin(derivat) als einphasige homogene Lösung vor. Es kommt nicht zu Ausfällungen und bleibt in Lösung. Die Infusionslösung kann zweckmäßig in Infusionsampullen abgefüllt werden.

Der Puffer für die erfindungsgemäße Infusionslösung ist vorzugsweise ein Citratpuffer, der insbesondere durch Zugabe von Citronensäure und Natriumcitrat bereitgestellt wird. Andere physiologisch verträgliche Puffersysteme sind ebenfalls denkbar. Wichtig ist die Einstellung und Stabilisierung des pH-Wertes der Infusionslösung im Bereich von 5,5 bis 6,0. Die Pufferbestandteile werden zweckmäßig in Mengen von 0,5 mg bis 5 mg pro 10 ml Infusionslösung verwendet, vorzugsweise von 0,5 mg bis 3 mg.

Die Konzentration an Curcumin(derivat) in der Infusionslösung liegt bevorzugt im Bereich von 100 bis 1000 mg pro 50 ml Lösung, insbesondere im Bereich von 250 bis 700 mg und ganz besonders bevorzugt bei etwa 500 mg pro 50 ml Lösung. Weiterhin sind in der Regel pro 50 ml Lösung folgende Komponenten enthalten:
4 g bis 17 g, vorzugsweise von 10 g bis 15 g Lösungsvermittler,
0,2 g bis 3 g, vorzugsweise von 0,5 g bis 2 g Dimethylsulfoxid,
100 bis 600 µg, vorzugsweise 100 bis 250 µg Natriumselenit oder Gemisch von Natriumselenit mit einem oder mehreren weiteren Antioxidantien und
2,5 mg bis 25 mg, vorzugsweise 4 mg bis 15 mg Puffer.

Die Infusionslösung wird bevorzugt aus dem erfindungsgemäßen Konzentrat hergestellt, indem das abgewogene Curcumin(derivat)-haltige Konzentrat dem, vorzugsweise auf etwa 60°C bis 70°C erwärmten, Infusionsmedium, beispielsweise Aqua ad injectabilia, langsam und unter Rühren zugegeben wird. Im nächsten Schritt wird der in demselben oder einem anderen Infusionsmedium, beispielsweise in Aqua ad injectabilia, gelöste Citratpuffer unter Rühren hinzugefügt. Die einphasige klare dunkelgelbe Lösung wird einige Zeit, beispielsweise 1 bis 30 min., vorzugsweise etwa 5 min., weitergerührt und, soweit das Infusionsmedium erwärmt wurde, auf Raumtemperatur abkühlen gelassen. Der gewünschte pH-Wert wird ggfs. ergänzend mit Citronensäure eingestellt und abschließend wird mit weiterem Infusionsmedium, beispielsweise Aqua ad injectabilia, auf das entsprechende Volumen aufgefüllt. Die gepufferte Lösung wird dann sterilisiert, z.B. indem sie sterilfiltriert und bei 121°C, 2013 mbar über 20 min. autoklaviert wird.

Alternativ kann die gewünschte Menge Curcumin(derivat) mit DMSO und Lösungsvermittler vermischt werden und anschließend erfolgt die Zugabe der weiteren Komponenten in beliebiger Reihenfolge, wobei jede der Komponenten auch zuerst mit Infusionsmedium vermischt werden kann und diese Mischung danach mit der Lösung von Curcumin(derivat) in DMSO vermischt wird. Die Mischung kann in jeder an sich bekannten Vorrichtung erfolgen.

Es versteht sich, dass sowohl alle Komponenten in der für Arzneimittel erforderlichen Reinheit eingesetzt werden als auch die Mischvorgänge in sterilen Vorrichtungen und unter den vorgeschriebenen Sterilitätsbedingungen durchgeführt werden.

Die erfindungsgemäße Infusionslösung ist stabil, gut verträglich und stellt das Curcumin(derivat) in einer direkt verfügbaren Form bereit. Die Infusion erfolgt in an sich bekannter Weise intravenös. Das Infusionsvolumen beträgt vorzugsweise von 100 bis 500 ml, z.B. 250 ml wenn 250 mg Curcumin(derivat) enthalten sind. Sofern vorhanden kann natürlich die Infusion über Verweilkatheter oder einen Portzugang administriert werden. Da die Lösung keinen Alkohol enthält können gängige Infusionssysteme eingesetzt werden. Alkoholhaltige Curcuminformulierungen dagegen bedürfen, wegen der chemischen Reaktion mit PVC-Infusionsbestecken, des Einsatzes PVC-freier Infusionssysteme. Es kann wie bereits erwähnt in einer hohen Geschwindigkeit infundiert werden. Selbst Raten von bis zu 15 ml/Minute sind gut vertragen worden. Als gut geeignet haben sich Infusionsgeschwindigkeiten von 4 bis 12 ml/Minute, bevorzugt von 8 bis 10 ml/Minute erwiesen.

Die Dosierung und Indikation für Curcumin sind an sich bekannt und werden vom Therapeuten festgelegt. Beispielsweise werden beim Vorliegen von chronisch manifestierten Entzündungen 125 mg bis 250 mg; beim Vorliegen von soliden Tumoren (ohne Metastasenbildung) 250 mg und bei Tumoren mit Fernabsiedlung von Metastasen 250 mg bis 500 mg Curcumin(derivat) pro Infusion verabreicht.

Dabei erfolgen in der Regel eine bis zwei Infusionen in 7 bis 14 Tagen.

Die Erfindung soll anhand der folgenden Beispiele erläutert werden, ohne jedoch auf die speziell beschriebenen Ausführungsformen beschränkt zu sein. Soweit nichts anderes angegeben ist oder sich aus dem Zusammenhang zwingend anders ergibt, beziehen sich Prozentangaben auf das Gewicht, im Zweifel auf das Gesamtgewicht der Mischung.

Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, dass zumindest um 10 % höhere oder niedrigere Werte oder um 5 % höhere oder niedrigere Werte und in jedem Fall um 1 % höhere oder niedrigere Werte eingeschlossen sind.

### Beispiel 1

250 mg Curcumin, extrahiert aus biologisch angebauter Curcuma longa, wurden bei Raumtemperatur in 0,5 ml sterilem DMSO gelöst. In dieser Lösung wurden 5,5 ml von durch Erwärmen auf etwa 60°C verflüssigtem Polyglycol mono- und diester von 12-Hydroxystearinsäure (Kolliphor HS 15, BASF SE, DE) dem gelösten Curcumin unter Rühren zugegeben. Aus der erhaltenen einphasigen Lösung entstand nach dem Abkühlen auf Raumtemperatur ein wasserfreies, lagerstabiles pastöses Konzentrat, aus dem sich durch Verdünnen mit Infusionsmedium unter Zusatz von Natriumselenit und Puffer eine erfindungsgemäße Infusionslösung erhalten lässt.

Beispiel 2250 mg Curcumin, extrahiert aus biologisch angebauter Curcuma longa, wurden bei Raumtemperatur in 0,5 ml sterilem DMSO gelöst. In dieser Lösung wurden 5,5 ml von durch Erwärmen auf etwa 60°C verflüssigtem Polyglycol mono- und diester von 12-Hydroxystearinsäure (Kolliphor HS 15, BASF SE, DE) dem gelösten Curcumin unter Rühren zugegeben. In dieser Lösung wurden 333 µg Natriumselenitpentahydrat (Na₂SeO₃ • 5 H₂O) entsprechend 100 µg Selen gelöst.

Aus der erhaltenen einphasigen Lösung entstand nach dem Abkühlen auf Raumtemperatur ein wasserfreies, lagerstabiles pastöses Konzentrat gemäß der Erfindung.

### Beispiel 3

500 mg Curcumin, extrahiert aus biologisch angebauter Curcuma longa, wurden bei Raumtemperatur in 1 ml sterilem DMSO gelöst. In dieser Lösung wurden 11 ml von durch Erwärmen auf etwa 60°C verflüssigtem Polyglycol mono- und diester von 12-Hydroxystearinsäure (Kolliphor HS 15, BASF SE, DE) dem gelösten Curcumin unter Rühren zugegeben. In dieser Lösung wurden 333 µg Natriumselenitpentahydrat (Na₂SeO₃ • 5 H₂O) entsprechend 100 µg Selen gelöst. Dann wurden 23 ml Aqua ad injectabilia unter Rühren zugegeben. Anschließend wurden 4,6 mg wasserfreie Citronensäure und 5 ml Natriumcitrat-Lösung 3,13 % (entsprechend 31,3 mg Natriumcitrat • 2H₂O / ml) unter Rühren hinzugefügt. Die klare dunkelgelbe Lösung wurde etwa 5 min. weitergerührt. Der gewünschte pH-Wert von 6,0 wurde mit 1,2 mg wasserfreier Citronensäure eingestellt und dann wurde mit Aqua ad injectabilia auf ein Volumen von 50 ml aufgefüllt. Die gepufferte Lösung wurde in Ampullen abgefüllt, darin sterilfiltriert und bei 121°C, 2013 mbar über 20 min. autoklaviert.

## Patentansprüche

1. Lösung für die intravenöse Infusion enthaltend:
Curcumin und/oder ein oder mehrere Curcuminderivate, nämlich Demethoxycurcumin und/oder Bisdemethoxycurcumin, Dimethylsulfoxid,
einen oder mehrere Lösungsvermittler,
Natriumselenit, und
einen Puffer
in einem wässrigen Infusionsmedium.

2. Lösung gemäß Anspruch 1, **dadurch gekennzeichnet, dass** von 100 mg bis 1000 mg Curcumin und/oder ein oder mehrere Curcuminderivate pro 50 ml Lösung enthalten sind, vorzugsweise von 300 mg bis 700 mg pro 50 ml Lösung.

3. Lösung gemäß Anspruch 1 oder 2, **dadurch gekennzeichnet, dass** der eine oder die mehrere Lösungsvermittler in einer Menge im Bereich von 15 g bis 25 g, vorzugsweise von 20 g bis 30 g, pro 1 g Curcumin und/oder dem einen oder mehreren Curcuminderivaten enthalten ist/sind.

4. Lösung gemäß einem der Ansprüche 1 bis 3, **dadurch gekennzeichnet, dass** der/die Lösungsvermittler ein oder mehrere solubilisierende Polymere ist/sind.

5. Lösung gemäß Anspruch 4, **dadurch gekennzeichnet, dass** der Lösungsvermittler ausgewählt ist aus der Gruppe bestehend aus Polyvinylpyrrolidon, Addukten von Ethylenoxid an physiologisch verträgliche Öle, Addukten von Propylenoxid an physiologisch verträgliche Öle, Polyethylenglykol, Polysorbat sowie Gemischen von zwei oder mehreren davon, besonders bevorzugt aus Polyethylenglykol, Polysorbat und Gemischen davon.

6. Lösung gemäß einem der Ansprüche 1 bis 5, **dadurch gekennzeichnet, dass** das wässrige Infusionsmedium steriles Wasser oder isotonische Kochsalzlösung oder Glukoselösung oder ein Gemisch davon ist.

7. Lösung gemäß einem der Ansprüche 1 bis 6, **dadurch gekennzeichnet, dass** Dimethylsulfoxid in einer Menge im Bereich von 2 g bis 6 g, vorzugsweise von 2 g bis 4 g, pro 1 g Curcumin und/oder dem einen oder mehreren Curcuminderivaten enthalten ist/sind

8. Lösung gemäß einem der Ansprüche 1 bis 7, **dadurch gekennzeichnet, dass** von 100 bis 600 µg, vorzugsweise von 100 bis 250 µg Natriumselenit pro 50 ml Lösung enthalten sind.

9. Lösung gemäß Anspruch 8, **dadurch gekennzeichnet, dass** ein weiteres Antioxidans oder mehrere weitere Antioxidantien enthalten sind, das/die vorzugsweise ausgewählt ist/sind unter Ascorbinsäure, N-Acetylcystein und Glutathion.

10. Lösung gemäß einem der Ansprüche 1 bis 9, **dadurch gekennzeichnet, dass** der Puffer aus Citronensäure und Natriumcitrat gebildet ist.

11. Lösung gemäß Anspruch 10, **dadurch gekennzeichnet, dass** Citronensäure und Natriumcitrat im Molverhältnis von 1:2 bis 2:1 in einer Menge von 2,5 mg bis 25 mg, vorzugsweise von 4 mg bis 15 mg, pro 50 ml Infusionslösung enthalten sind.

12. Lösung gemäß einem der Ansprüche 1 bis 11, **dadurch gekennzeichnet, dass** pro 50 ml Lösung:
100 mg bis 1000 mg, vorzugsweise 300 mg bis 700 mg und besonders bevorzugt etwa 500 mg Curcumin und/oder ein oder mehrere Curcuminderivate
4 g bis 17 g, vorzugsweise von 10 g bis 15 g Lösungsvermittler,
0,2 g bis 3 g, vorzugsweise von 0,5 g bis 2 g Dimethylsulfoxid,
100 bis 600 µg, vorzugsweise 100 bis 250 µg Natriumselenit und
2,5 mg bis 25 mg, vorzugsweise 4 mg bis 15 mg Puffer enthalten sind.

13. Konzentrat zur Herstellung wässriger Lösungen für die intravenöse Infusion enthaltend Curcumin und/oder ein oder mehrere Curcuminderivate, nämlich Demethoxycurcumin und/oder Bisdemethoxycurcumin, Dimethylsulfoxid, einen oder mehrere Lösungsvermittler und Natriumselenit.

14. Konzentrat gemäß Anspruch 13, **dadurch gekennzeichnet, dass** Natriumselenit in Mengen von 0,1 bis 0,6 mg, vorzugsweise von 0,1 bis 0,25 mg, pro 1 g Curcumin und/oder ein oder mehrere Curcuminderivate(n) enthalten ist.

15. Konzentrat gemäß Anspruch 13 oder 14, **dadurch gekennzeichnet, dass** Dimethylsulfoxid in einer Menge im Bereich von 2 g bis 6 g, vorzugsweise 2 g bis 4 g, pro 1 g Curcumin und/oder ein oder mehrere Curcuminderivate(n) enthalten ist.

16. Konzentrat gemäß einem der Ansprüche 13 bis 15, **dadurch gekennzeichnet, dass** der eine oder die mehreren Lösungsvermittler in einer Menge im Bereich von 15 g bis 25 g, vorzugsweise von 20 g bis 30 g, pro 1 g Curcumin und/oder dem einen oder mehreren Curcuminderivaten enthalten ist/sind.

## Claims

1. Solution for intravenous infusion containing:
curcumin and/or one or more curcumin derivatives, namely demethoxycurcumin and/or bisdemethoxycurcumin, dimethyl sulfoxide,
one or more solubilizers,
sodium selenite, and
a buffer
in an aqueous infusion medium.

2. Solution according to claim 1, **characterized in that** from 100 mg to 1000 mg curcumin and/or one or more curcumin derivatives are contained per 50 ml solution, preferably from 300 mg to 700 mg per 50 ml solution.

3. Solution according to claim 1 or 2, **characterized in that** the one or more solubilizers is/are contained in an amount ranging from 15 g to 25 g, preferably from 20 g to 30 g, per 1 g of curcumin and/or the one or more curcumin derivatives.

4. Solution according to any one of claims 1 to 3, **characterized in that** the solubilizer(s) is/are one or more solubilizing polymers.

5. Solution according to claim 4, **characterized in that** the solubilizer(s) is/are selected from the group consisting of polyvinylpyrrolidone, adducts of ethylene oxide to physiologically compatible oils, adducts of propylene oxide to physiologically compatible oils, polyethylene glycol, polysorbate, and mixtures of two or more thereof, particularly preferred polyethylene glycol, polysorbate, and mixtures thereof.

6. Solution according to any one of claims 1 to 5, **characterized in that** the aqueous infusion medium is sterile water or isotonic saline or glucose solution or a mixture thereof.

7. Solution according to any one of claims 1 to 6, **characterized in that** dimethyl sulfoxide is contained in an amount in the range of from 2 g to 6 g, preferably from 2 g to 4 g, per 1 g of curcumin and/or the one or more curcumin derivatives

8. Solution according to any one of claims 1 to 7, **characterized in that** from 100 to 600 µg, preferably from 100 to 250 µg, sodium selenite are contained per 50 ml of solution.

9. Solution according to claim 8, **characterized in that** one or more further antioxidant(s) are included, which are preferably selected from ascorbic acid, N-acetylcysteine and glutathione.

10. Solution according to any one of claims 1 to 9, **characterized in that** the buffer is formed from citric acid and sodium citrate.

11. Solution according to claim 10, **characterized in that** citric acid and sodium citrate are contained in a molar ratio of from 1:2 to 2:1 in an amount from 2.5 mg to 25 mg, preferably from 4 mg to 15 mg, per 50 ml of infusion solution.

12. Solution according to any one of claims 1 to 11, **characterized in that** it contains per 50 ml of solution:
100 mg to 1000 mg, preferably 300 mg to 700 mg and particularly preferred about 500 mg of curcumin and/or one or more curcumin derivatives,
4 g to 17 g, preferably from 10 g to 15 g, solubilizer,
0.2 g to 3 g, preferably from 0.5 g to 2 g, dimethyl sulfoxide,
100 to 600 µg, preferably 100 to 250 µg, sodium selenite, and
2.5 mg to 25 mg, preferably 4 mg to 15 mg, buffer.

13. Concentrate for the preparation of aqueous solutions for intravenous infusion containing curcumin and/or one or more curcumin derivatives, namely demethoxycurcumin and/or bisdemethoxycurcumin, dimethyl sulfoxide, one or more solubilizers and sodium selenite.

14. Concentrate according to claim 13, **characterized in that** sodium selenite is present in amounts of from 0.1 to 0.6 mg, preferably from 0.1 to 0.25 mg, per 1 g of curcumin and/or one or more curcumin derivatives.

15. Concentrate according to claim 13 or 14, **characterized in that** dimethyl sulfoxide is present in an amount ranging from 2 g to 6 g, preferably from 2 g to 4 g, per 1 g of curcumin and/or one or more curcumin derivative(s).

16. Concentrate according to any one of claims 13 to 15, **characterized in that** the one or more solubilizers is/are present in an amount ranging from 15 g to 25 g, preferably from 20 g to 30 g, per 1 g of curcumin and/or the one or more curcumin derivatives.

## Revendications

1. Solution pour la perfusion intraveineuse contenant :
de la curcumine et/ou un ou plusieurs dérivés de curcumine, à savoir de la déméthoxycurcumine et/ou de la bisdéméthoxycurcumine, du diméthylsulfoxyde,
un ou plusieurs agents de solubilisation,
du sélénite de sodium, et
un tampon
dans un milieu de perfusion aqueux.

2. Solution selon la revendication 1, **caractérisée en ce que** de 100 mg à 1 000 mg de curcumine et/ou d'un ou de plusieurs dérivés de curcumine sont contenus pour 50 mL de solution, de préférence de 300 mg à 700 mg pour 50 mL de solution.

3. Solution selon la revendication 1 ou la revendication 2, **caractérisée en ce que** les un ou plusieurs agents de solubilisation sont contenus dans une quantité comprise dans la gamme de 15 g à 25 g, de préférence de 20 g à 30 g, pour 1 g de curcumine et/ou des un ou plusieurs dérivés de curcumine.

4. Solution selon l'une des revendications 1 à 3, **caractérisée en ce que** le ou les agents de solubilisation sont un ou plusieurs polymères solubilisants.

5. Solution selon la revendication 4, **caractérisée en ce que** l'agent de solubilisation est sélectionné dans le groupe constitué de la polyvinylpyrrolidone, d'adduits d'oxyde d'éthylène sur des huiles physiologiquement acceptables, d'adduits d'oxyde de propylène sur des huiles physiologiquement acceptables, du polyéthylène glycol, du polysorbate ainsi que de mélanges de deux de ceux-ci ou plus, de manière particulièrement préférée du polyéthylène glycol, du polysorbate et de mélanges de ceux-ci.

6. Solution selon l'une des revendications 1 à 5, **caractérisée en ce que** le milieu de perfusion aqueux est de l'eau stérile ou de la solution saline isotonique ou de la solution de glucose ou un mélange de celles-ci.

7. Solution selon l'une des revendications 1 à 6, **caractérisée en ce que** le diméthylsulfoxyde est contenu dans une quantité comprise dans la gamme de 2 g à 6 g, de préférence de 2 g à 4 g, pour 1 g de curcumine et/ou des un ou plusieurs dérivés de curcumine.

8. Solution selon l'une des revendications 1 à 7, **caractérisée en ce que** de 100 à 600 µg, de préférence de 100 à 250 µg de sélénite de sodium sont contenus pour 50 mL de solution.

9. Solution selon la revendication 8, **caractérisée en ce qu'**un antioxydant supplémentaire ou plusieurs antioxydants supplémentaires sont contenus, lequel ou lesquels étant sélectionnés de préférence parmi l'acide ascorbique, la N-acétylcystéine et le glutathion.

10. Solution selon l'une des revendications 1 à 9, **caractérisée en ce que** le tampon est formé à partir d'acide citrique et de citrate de sodium.

11. Solution selon la revendication 10, **caractérisée en ce que** l'acide citrique et le citrate de sodium sont contenus dans le rapport molaire de 1:2 à 2:1 dans une quantité de 2,5 mg à 25 mg, de préférence de 4 mg à 15 mg, pour 50 mL de solution de perfusion.

12. Solution selon l'une des revendications 1 à 11, **caractérisée en ce que**, pour 50 mL de solution, sont contenus :
de 100 mg à 1 000 mg, de préférence de 300 mg à 700 mg et de manière particulièrement préférée environ 500 mg de curcumine et/ou d'un ou de plusieurs dérivés de curcumine,
de 4 g à 17 g, de préférence de 10 g à 15 g d'agent de solubilisation,
de 0,2 g à 3 g, de préférence de 0,5 g à 2 g de diméthylsulfoxyde,
de 100 à 600 µg, de préférence de 100 à 250 µg de sélénite de sodium et
de 2,5 mg à 25 mg, de préférence de 4 mg à 15 mg de tampon.

13. Concentré pour la préparation de solutions aqueuses pour la perfusion intraveineuse contenant de la curcumine et/ou un ou plusieurs dérivés de curcumine, à savoir de la déméthoxycurcumine et/ou de la bisdéméthoxycurcumine, du diméthylsulfoxyde, un ou plusieurs agents de solubilisation et du sélénite de sodium.

14. Concentré selon la revendication 13, **caractérisé en ce que** le sélénite de sodium est contenu dans des quantités de 0,1 à 0,6 mg, de préférence de 0,1 à 0,25 mg, pour 1 g de curcumine et/ou d'un ou de plusieurs dérivés de curcumine.

15. Concentré selon la revendication 13 ou la revendication 14, **caractérisé en ce que** le diméthylsulfoxyde est contenu dans une quantité comprise dans la gamme de 2 g à 6 g, de préférence de 2 g à 4 g, pour 1 g de curcumine et/ou d'un ou de plusieurs dérivés de curcumine.

16. Concentré selon l'une des revendications 13 à 15, **caractérisé en ce que** les un ou plusieurs agents de solubilisation sont contenus dans une quantité comprise dans la gamme de 15 g à 25 g, de préférence de 20 g à 30 g, pour 1 g de curcumine et/ou des un ou plusieurs dérivés de curcumine.
